# Europäisches Patentamt

## European Patent Office

## Office européen des brevets

(11) Veröffentlichungsnummer: **0 005 235**
**A2**

(12) **EUROPÄISCHE PATENTANMELDUNG**

(21) Anmeldenummer: 79101245.3

(22) Anmeldetag: 25.04.79

(51) Int. Cl.²: **C 02 B 3/02,** C 02 B 1/34, **A 61 L 3/00,** A 61 L 1/00

(30) Priorität: 27.04.78 DE 2818412

(43) Veröffentlichungstag der Anmeldung: 14.11.79
Patentblatt 79/23

(84) Benannte Vertragsstaaten: BE CH DE FR GB IT NL SE

(71) Anmelder: **Ihle Ingenieurgesellschaft mbH, Am Hackenbruch 45, D-4000 Düsseldorf (DE)**

(72) Erfinder: **Albersmeyer, Walter, Dr., Grotenbachstrasse 74, D-5270 Gummersbach 1 (DE)**
Erfinder: **Liebig, Rolf, Däinghauser Strasse4, D-5277 Marienheide (DE)**
Erfinder: **Weigand, Friedrich, Luegallee 102, D-4000 Düsseldorf 11 (DE)**

(74) Vertreter: **Cohausz, Werner, Dipl.-Ing. et al, Schumannstrasse 97, D-4000 Düsseldorf (DE)**

(54) **Vorrichtung zur Desinfektion und/oder zum Entalgen von Wasser.**

(57) Die Erfindung betrifft eine Vorrichtung zur Desinfektion oder zum Entalgen von Wasser durch ultraviolette Strahlung, bei der das Halogen oder der Sauerstoff vor seinem Austreten in das Wasser eine in unmittelbarer Nähe der Strahlenquelle angeordnete Kammer oder Leitung durchströmt, deren Inneres von ultravioletten Strahlen durchdrungen wird, um die Wirkung des Halogens oder des Sauerstoffs zu verstärken.

Anm.: Ihle Ingenieurgesellschaft mbH
Am Hackenbruch 45, 4000 Düsseldorf

Vorrichtung zur Desinfektion und/oder
zum Entalgen von Wasser

Die Erfindung betrifft eine Vorrichtung zur Desinfektion
und/oder zum Entalgen von sauerstoff- und/oder halogen-,
insbesondere chlorhaltigem Wasser durch ultraviolette
Strahlung, wobei vor einer intensiven Vermischung des
einzuführenden Halogens und/oder Sauerstoffs mit dem
Wasser das Halogen und/oder der Sauerstoff in einen unmittelbar nahen Bereich zur Strahlenquelle gebracht wird.

Es ist bekannt, in das Wasser von Schwimmbecken Chlor
einzugeben und zusätzlich dieses Wasser durch eine Kammer
zu leiten, in der es einer UV-Strahlung ausgesetzt ist
(DT-OS 25 43 418). Bei diesen bekannten Vorrichtungen hat
es sich gezeigt, daß verhältnismäßig hohe Chlormengen in
das Wasser eingebracht werden müssen, um eine ausreichende Desinfektion zu erzielen.

Ferner ist aus der US-PS 3 924 139 eine Vorrichtung bekannt, bei der sauerstoffhaltiges Gas in der Nähe einer
UV-Strahlenquelle in Abwasser eingebracht wird, um die
Oxidationsreaktion zu unterstützen und Schmutzablagerungen
auf der Strahlenquelle zu verhindern. Aufgrund des wechselnden Trübungsgrades des Abwassers wird dabei das Gas

32 125
HC/Os

unterschiedlich stark durchstrahlt. Ferner ist das Gas,
während es sich in der Nähe der Strahlenquelle befindet,
perl- oder bläschenförmig, wobei ein Teil der Gasbläschen durch näherliegende abgedeckt werden und dadurch
nur ungenügend durchstrahlt werden. Auch findet hier
eine gründliche Durchmischung des Gases mit dem Abwasser
nicht statt.

Aufgabe der Erfindung ist es, eine Vorrichtung der eingangs genannten Art derart zu verbessern, daß eine gleichmäßig starke genau bestimmte Dissoziation des Gases erreicht wird.

Darüberhinaus ist es Aufgabe der Erfindung, eine Vorrichtung der eingangs genannten Art derart zu verbessern, daß
bei einem hohen Desinfektionsgrad nur geringe Halogen-
bzw. Chlormengen benötigt werden.

Diese Aufgaben werden erfindungsgemäß dadurch gelöst, daß
das Halogen und/oder der Sauerstoff vor seinem Austreten
in das Wasser eine in unmittelbarer Nähe der Strahlenquelle angeordnete Kammer oder Leitung durchströmt, deren
Inneres von Strahlen durchdrungen wird.                    —

Bei der erfindungsgemäßen Vorrichtung werden die Halogen-
bzw. Chlormoleküle, ehe sie mit dem Wasser vermischt werden,
ausreichend und gleichmäßig gespalten und wirken dadurch
erheblich intensiver auf das Wasser desinfizierend ein.
Es werden bei einem gleich hohen Desinfektionsgrad nur
geringe Halogen- bzw. Chlormengen benötigt, so daß das
Wasser nur gering belastet wird. Alternativ läßt sich bei
einer gleich starken Belastung des Wassers ein wesentlich
höherer Desinfektionsgrad erreichen. Der Trübungsgrad des
Wassers hat auf den Dissoziationsgrad des Gases keinen
Einfluß, so daß eine exakte Dosierung und ein vorherbestimmbarer Behandlungsgrad ermöglicht wird.

Ein nahes Heranführen des Halogens an die Strahlenquelle
führt darüberhinaus zu dem Vorteil, daß die Temperatur
des Halogens erhöht und dadurch die Dissoziation verstärkt wird.

Vorzugsweise wird vorgeschlagen, daß nach der Durchmischung eines Wasserstromes mit dissoziiertem Halogen
dieser Wasserstrom mit einem zweiten zu behandelnden
Wasserstrom vermischt wird. Durch ein derartiges Verfahren kann nicht nur ein Wasserstrom in zwei aufgeteilt
werden, wobei der erstere zuerst mit dissoziiertem Halogen durchmischt wird und danach den zweiten desinfiziert,
sondern es können auch verschieden stark mit Keimen versehene Wasserströme desinfiziert werden, indem vorzugsweise zuerst der stärker verkeimte Wasserstrom behandelt
und danach mit dem weniger verkeimten vermischt wird.

Eine sehr einfache Konstruktion wird dadurch geschaffen,
wenn die Strahlenquelle teilweise oder in einem Abschnitt
von einer Wandung umgeben ist und zwischen der Wandung
und der Außenwand der Strahlenquelle die Austrittsöffnung für das Halogen angeordnet ist.

Zusätzlich oder alternativ kann aber auch in Nähe der
Strahlenquelle eine strahlendurchlässige Leitung oder
Röhre angeordnet sein, durch die das Halogen vor seinem
Austritt ins Wasser strömt. In diesem letzteren Fall kann
die Leitung oder Röhre die Strahlenquelle koaxial umgeben,
oder die Leitung oder Röhre längs der Strahlenquelle angeordnet sein oder die Strahlenquelle umwinden.

Eine ausreichende Durchmischung des Wassers mit Halogen als auch eine ständig gleichbleibend starke UV-Strahlung wird dadurch erreicht, daß jede Strahlenquelle von einem Quarzschutzrohr umgeben ist, an dem Leitbleche wendelförmig anliegen, die zur Reinigung der Quarzrohroberfläche längs des Quarzrohres insbesondere motorisch bewegbar sind.

Eine sehr schnelle und wirksame Durchmischung kann dadurch erreicht werden, daß die Strahlenquelle vor oder innerhalb eines Venturirohres angeordnet ist. Ferner kann die erfindungsgemäße Vorrichtung am oder im Ein- oder Austritt einer Pumpe angeordnet sein, wodurch die Vorrichtung nicht nur sehr platzsparend untergebracht ist, sondern auch eine sehr wirksame Durchmischung erreicht wird.

Ausführungsbeispiele der Erfindung sind in den Zeichnungen dargestellt und werden im folgenden näher beschrieben. Es zeigen:

Fig. 1     einen Schnitt durch eine Vorrichtung
            mit zusätzlichem Bypass;

Fig. 2        einen Schnitt durch eine alternative Vorrichtung mit angeschlossener Regeleinrichtung;

Fig. 3        eine in einem Venturirohr angeordnete Vorrichtung;

Fig. 4        eine Anordnung einer Vorrichtung an einer Zusammenführung zweier Wasserströme;

Fig. 5        eine im Auslaß einer Kreiselpumpe angeordnete Vorrichtung;

Fig. 6        einen Schnitt entlang der Linie A-A in Fig. 1 in größerem Maßstab; und

Fig.7-10     unterschiedliche Anordnungen der das Halogen oder den Sauerstoff zuführenden Leitungen.

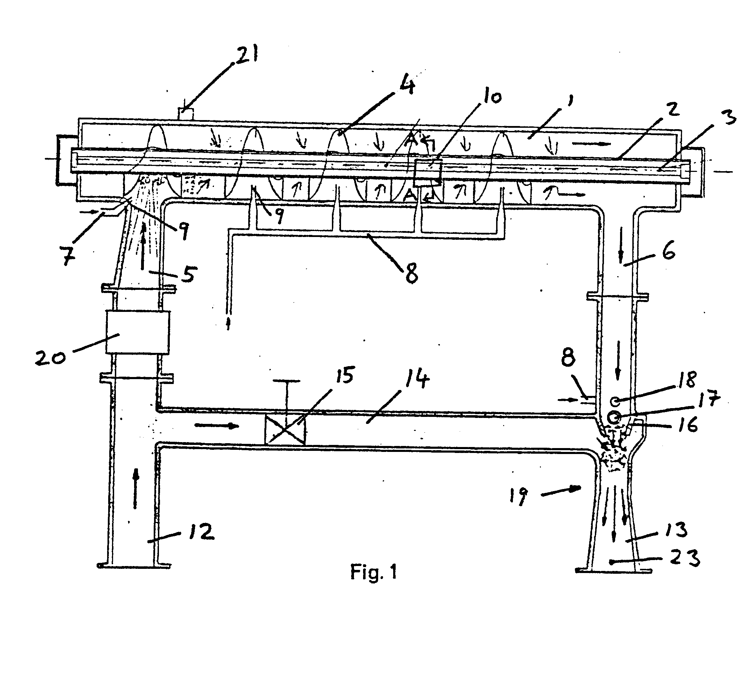

Nach Fig. 1 ist eine Behandlungskammer 1 von einem an beiden Stirnseiten verschlossenen Rohrabschnitt gebildet, in dem koaxial ein Quarzschutzrohr 2 über die gesamte Länge einliegt, wobei das Quarzschutzrohr die Stirnseiten durchdringt und dort abgedichtet ist. In dem Quarzschutzrohr liegt eine röhrenförmige UV-Lampe 3 ein, die zur Erzeugung der UV-Strahlung Antimon verdampft. Das Quarzschutzrohr ist wendelförmig oder schraubenförmig von einem das Wasser verwirbelnden Leitblech 4 umgeben, das den Raum zwischen dem Quarzschutzrohr 2 und der Innenwandung des Rohrabschnitts der Behandlungskammer 1 ausfüllt und nicht nur für das Quarzschutzrohr 2 eine stützende Funktion besitzt, sondern axial verschoben werden kann und aufgrund seiner Anlage an der Außenseite des Quarzschutzrohres 2 dieses hierdurch reinigt. Die axiale Bewegung des Leitblechs 4 bzw. mehrerer Leitblechabschnitte wird durch
ein achsparalleles, nicht gezeigtes Gestänge erreicht.

In der Nähe der einander gegenüberliegenden Stirnseiten der
Behandlungskammer 1 ist jeweils ein Einlaß 5 und ein Auslaß 6
angeordnet. Das Chlor wird über eine in Nähe des Einlasses 5
angeordnete Leitung 7 und eine zwischen Einlaß und Auslaß angeordnete Leitung 8 zugeführt, wobei die Austrittsmündungen
dieser Leitungen in Nähe des Quarzschutzrohres 2 angeordnet
und als Düsen 9 ausgebildet sind. Das aus diesen Düsen 9 austretende Chlor ist sofort nach seinem Austritt einer intensiven UV-Strahlung ausgesetzt und wird dadurch sofort dissoziiert, ehe es mit dem Wasser intensiv vermischt wird. Ein
besonders langes Verweilen des Chlors in Nähe der Strahlenquelle bzw. des Quarzschutzrohres 2 wird dadurch erreicht, daß
die Austrittsöffnungen der Chlorleitungen 8 bzw. die Düsen 9
von einer zylinderabschnittförmigen Fläche 10 umgeben sind, die
koaxial in einem Abstand zum Quarzschutzrohr 2 angeordnet sind,

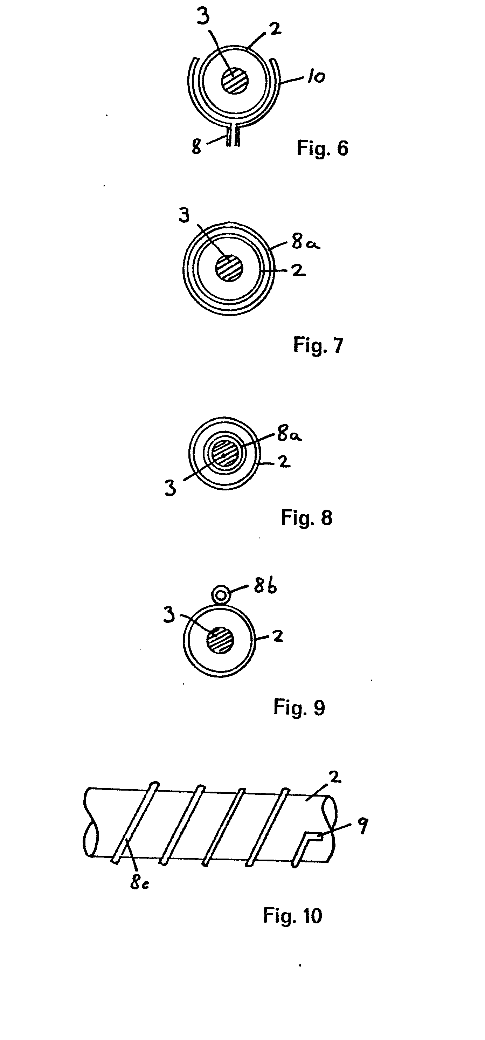

Fig. 1 und Fig. 6. Dadurch tritt das Chlor zuerst in den Raum
zwischen der Fläche 10 und dem Quarzschutzrohr 2 aus, bleibt
dort einen kurzen Zeitraum und tritt danach an den Rändern der
Fläche 10 aus, um intensiv mit dem Wasser vermischt zu werden.

Ein hoher Dissoziationsgrad kann auch dadurch erreicht werden,
daß die Leitung 8a aus UV-strahlendurchlässigem Material besteht und das Quarzschutzrohr (Fig. 7) oder das Rohr der
Leuchtstofflampe (Fig. 8) zumindest über einen bestimmten
axialen Abschnitt koaxial umgibt und damit einen ringförmigen
Querschnitt aufweist, oder daß die durchlässige Leitung 8b
an dem Quarzschutzrohr 2 längs befestigt ist (Fig. 9) oder
das Quarzschutzrohr in Windungen 9c umgibt (Fig. 10).

Am Einlaß 5 und Auslaß 6 ist jeweils eine Einlaufleitung 12 und eine Auslaufleitung 13 befestigt, die miteinander durch einen Bypass 14 verbunden sind, in dem ein Schieber 15 angeordnet ist. Der Bypass 14 mündet in der Auslaufleitung 13 an einer Stelle, an der das von der Behandlungskammer 1 kommende Wasser über eine Querschnittsverengung 16 dem Bypassstrom zugeführt wird, wobei in der Querschnittsverengung 16 eine weitere UV-Strahlenquelle 17 und in Strömungsrichtung vor der Strahlenquelle eine Chlormündung 18 angeordnet ist. Der von dem Auslaß 6 kommende Wasserstrom wird damit noch einmal behandelt und danach dem vom Einlauf 12 abzweigenden Strom beigemischt. Die Vermischung beginnt am Einlaß eines Venturirohrs 19, das den Auslauf 13 bildet. Die Vorrichtung kann im Filterkreislauf eines Schwimmbeckens angeordnet sein, wobei eine zwischen der Einlaufleitung 12 und dem Einlaß 5 angeordnete Pumpe 20 das Umwälzen des Wassers erzeugt.

In Nähe des Einlasses 5 ist in der Wandung der Behandlungskammer 1 eine Meßstelle 21 angeordnet, die die UV-Strahlendurchlässigkeit des Wassers mißt, diesen Wert einer Regeleinrichtung 22 eingibt, die die Chlorzufuhr bestimmt. Die Meßstelle 21 kann auch im Einlaß 5 der Behandlungskammer angeordnet sein. Ferner ist in der Auslaufleitung 13 eine Meßstelle 23 angeordnet, die den Resthalogengehalt bestimmt und diesen Wert zusätzlich der Regeleinrichtung 22 eingibt, so daß auch durch diesen Parameter die Chlorzugabe bestimmt wird.

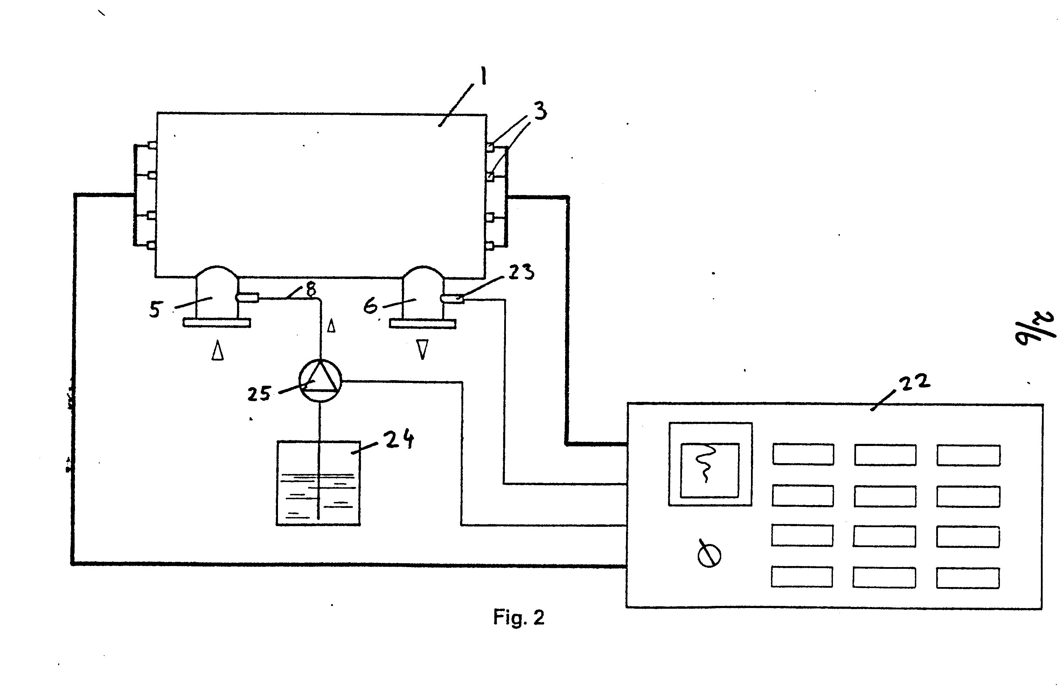

Das Chlor wird der Behandlungskammer von einem Chlorbehälter 24 über eine Dosierpumpe 25 zugeführt, die von der Regeleinrichtung 22 gesteuert wird. Die Behandlungskammer kann verhältnismäßig kurz ausgeführt sein, wenn statt einer Strahlenquelle mehrere, wie in Fig. 2 zu sehen, zueinander, parallel in der Kammer angeordnet sind.

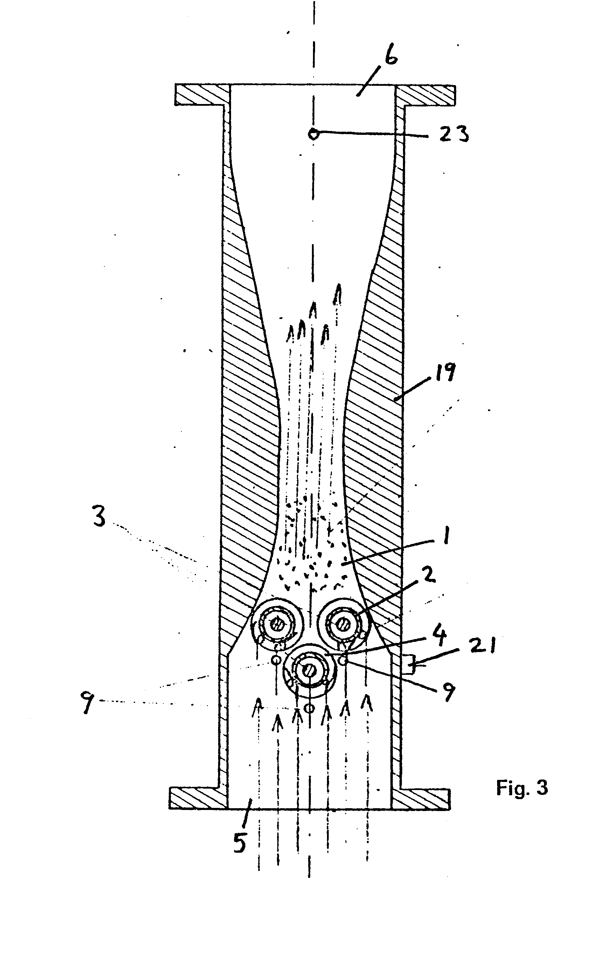

Bei dem in Fig. 3 gezeigten Ausführungsbeispiel ist ein Rohrabschnitt zu einem Venturirohr 19 ausgebildet, an dessen Einlaß drei UV-Strahlenquellen 3 rechtwinklig zur Venturirohrlängsachse angeordnet sind. Jeder Strahlenquelle ist nahe anliegend die Mündung oder die Düse 9 einer Chlorleitung vorgeordnet, und in Nähe der UV-Strahlenquellen befindet sich eine Meßstelle 21 zur Bestimmung der UV-Strahlendurchlässigkeit des Wassers. Ferner ist im Auslaß des Venturirohrs 19 eine Meßstelle 23 angeordnet, die den Desinfektionsgrad mißt.

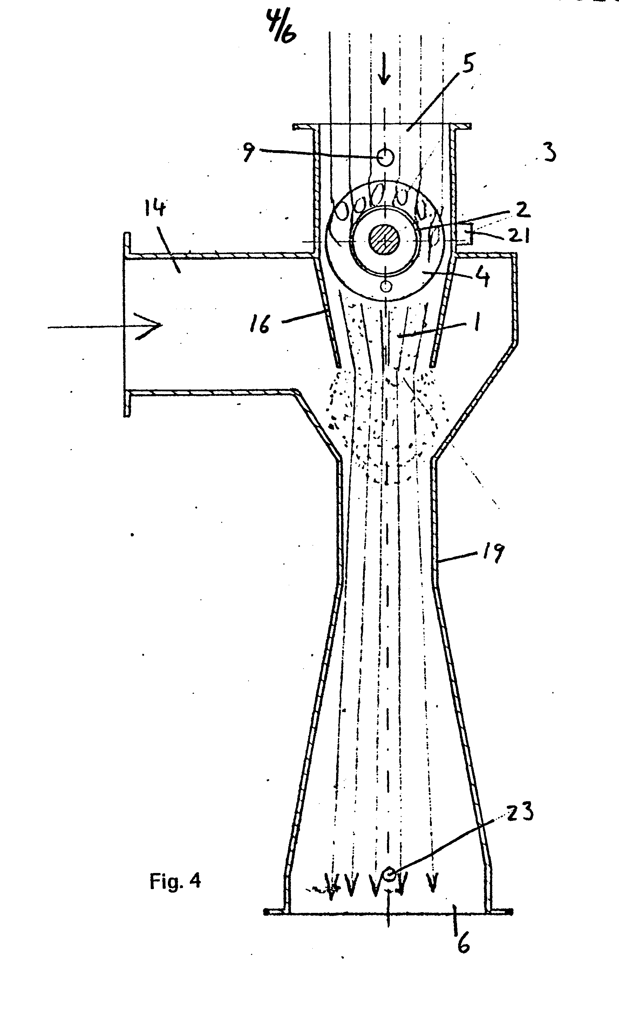

Fig. 4 zeigt in vergrößertem Maßstab die in Fig. 1 dargestellte Zusammenführung des Bypass 14 mit der von der Behandlungskammer kommenden Strömung. Dabei ist eine Meßstelle 21 zur Bestimmung der UV-Strahlendurchlässigkeit des Wassers in Höhe des Quarzschutzrohres 2 in der Wandung des Auslaßrohres 6 angeordnet.

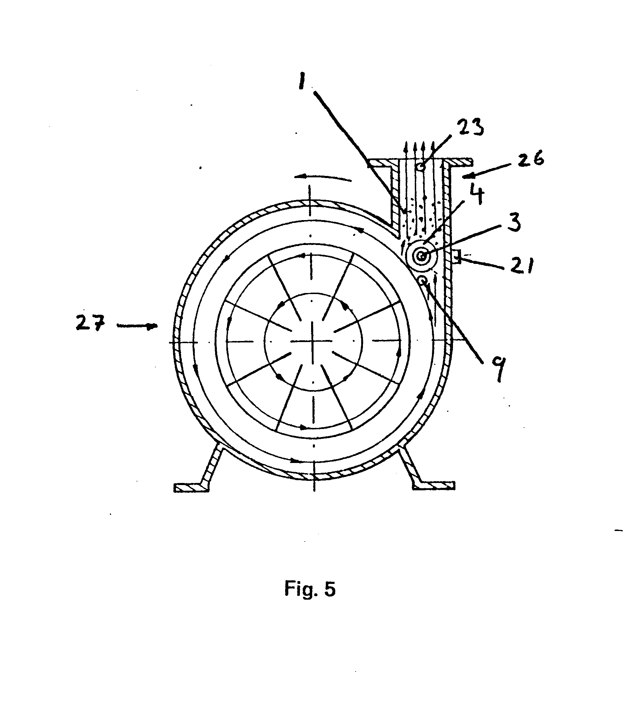

Entsprechend Fig. 5 bildet der Auslaß 26 einer Kreiselpumpe 27 eine Behandlungskammer, in der koaxial zum Läufer der Pumpe wenigstens eine Strahlenquelle 3 angeordnet ist. Wiederum befindet sich in Strömungsrichtung vor der Strahlenquelle in nächster Nähe dieser eine Mündung oder Düse 9 für eine Chlorleitung, und ferner ist in Höhe der Strahlenquelle eine Meß- stelle 21 und in Strömungsrichtung hinter der Strahlenquelle am Ende der Behandlungskammer eine Meßstelle 23 angeordnet.

# COHAUSZ & FLORACK

PATENTANWALTSBÜRO

SCHUMANNSTR. 97 · D-4000 DÜSSELDORF

Telefon: (02 11) 68 33 46          Telex: 0858 6513 cop d

0005235

PATENTANWÄLTE:

Dipl.-Ing. W. COHAUSZ · Dipl.-Ing. R. KNAUF · Dr.-Ing., Dipl.-Wirtsch.-Ing. A. GERBER · Dipl.-Ing. H. B. COHAUSZ

## Ansprüche

1. Vorrichtung zur Desinfektion und/oder zum Entalgen von sauerstoff- und/oder halogen-, insbesondere chlorhaltigem Wasser durch ultraviolette Strahlung, wobei vor einer intensiven Vermischung des einzuführenden Halogens und/oder Sauerstoffs mit dem Wasser das Halogen und/oder der Sauerstoff in einen unmittelbar nahen Bereich zur Strahlenquelle gebracht wird, d a d u r c h g e k e n n z e i c h n e t , daß das Halogen und/oder der Sauerstoff vor seinem Austreten in das Wasser eine in unmittelbarer Nähe der Strahlenquelle angeordnete Kammer oder Leitung durchströmt, deren Inneres von Strahlen durchdrungen wird.

2. Vorrichtung nach Anspruch 1, d a d u r c h g e - k e n n z e i c h n e t, daß das Halogen und/oder der Sauerstoff in unmittelbarer Nähe der Strahlenquelle (3) in das Wasser eingebracht wird.

3. Vorrichtung nach Anspruch 1 oder 2, d a d u r c h g e k e n n z e i c h n e t, daß in der Vorrichtung zwei Wasserströme gebildet werden, dessen erster nach der Durchmischung mit dissoziiertem Halogen bzw. Sauerstoff mit dem zweiten zu behandelnden Wasserstrom (14) vermischt wird.

32 125

HC/Os                    - 2 -

4. Vorrichtung nach einem der Ansprüche 1 bis 3, d a -
d u r c h  g e k e n n z e i c h n e t, daß die
Strahlenquelle teilweise oder in einem Abschnitt
von einer Wandung umgeben ist und zwischen der Wandung und der Außenwand der Strahlenquelle die Austrittsöffnung für das Halogen und/oder den Sauerstoff
angeordnet ist.

5. Vorrichtung nach einem der Ansprüche 1 bis 3, d a -
d u r c h  g e k e n n z e i c h n e t, daß in Nähe
der Strahlenquelle eine strahlendurchlässige Leitung
oder Röhre angeordnet ist, durch die das Halogen und/
oder der Sauerstoff vor seinem Austritt ins Wasser
strömt.

6. Vorrichtung nach Anspruch 5, d a d u r c h  g e -
k e n n z e i c h n e t, daß die Leitung oder Röhre
die Strahlenquelle koaxial umgibt.

7. Vorrichtung nach Anspruch 5, d a d u r c h  g e -
k e n n z e i c h n e t, daß die Leitung oder Röhre
längs der Strahlenquelle angeordnet ist.

8. Vorrichtung nach Anspruch 5, d a d u r c h  g e -
k e n n z e i c h n e t, daß die Leitung oder Röhre
die Strahlenquelle umwindet.

9. Vorrichtung nach einem der Ansprüche 4 bis 8, d a -
d u r c h  g e k e n n z e i c h n e t, daß jede
Strahlenquelle (3) von einem Quarzschutzrohr (2) umgeben ist, an dem zur Turbulenzerzeugung Leitbleche
(4) insbesondere wendelförmig anliegen, die zur Reinigung der Quarzrohroberfläche längs Quarzrohres insbesondere motorisch bewegbar sind.

0005235

lo. Vorrichtung nach einem der Ansprüche 4 bis 9, d a -
    d u r c h   g e k e n n z e i c h n e t, daß die
    Strahlenquelle (3) vor oder innerhalb eines Venturirohres (19) angeordnet ist.

11. Vorrichtung nach einem der Ansprüche 4 bis lo, d a -
    d u r c h   g e k e n n z e i c h n e t, daß sie am
    oder im Ein- oder Austritt einer Pumpe (27) angeordnet
    ist.

Fig. 1

Fig. 2

Fig. 3

Fig. 4

Fig. 5

0005235

Fig. 6

Fig. 7

Fig. 8

Fig. 9

Fig. 10